**Europäisches Patentamt**

**European Patent Office**

**Office européen des brevets**

(11) Veröffentlichungsnummer: **0 041 696**

**A1**

(12) # EUROPÄISCHE PATENTANMELDUNG

(21) Anmeldenummer: **81104284.5**

(22) Anmeldetag: **04.06.81**

(51) Int. Cl.³: **A 61 B 5/02**

(30) Priorität: **10.06.80 DE 3021658**

(43) Veröffentlichungstag der Anmeldung:
**16.12.81 Patentblatt 81/50**

(84) Benannte Vertragsstaaten:
**AT BE CH FR GB IT LI LU NL SE**

(71) Anmelder: **Rieckmann, Jutta, Geb. Federlein**
**Hauptstrasse 12**
**D-6719 Altleiningen(DE)**

(71) Anmelder: **Rieckmann, Peter, Dr.**
**Hauptstrasse 12**
**D-6719 Altleiningen(DE)**

(72) Erfinder: **Rieckmann, Jutta, Geb. Federlein**
**Hauptstrasse 12**
**D-6719 Altleiningen(DE)**

(72) Erfinder: **Rieckmann, Peter, Dr.**
**Hauptstrasse 12**
**D-6719 Altleiningen(DE)**

(74) Vertreter: **Ratzel, Gerhard, Dr.**
**Seckenheimer Strasse 36a**
**D-6800 Mannheim 1(DE)**

(54) **Vorrichtung zur Blutdruckmessung.**

(57) Die Erfindung betrifft eine Vorrichtung zur Blutdruckmessung mit einer am Arm zu befestigenden Manschette, (15) die einen Pulsfühler (18) aufweist, einer an die Manschette (15) angeschlossenen Druckquelle (25) und einem Druckmeßgerät (29) zur Messung des Druckes in der Manschette, (15) wobei erfindungsgemäß die Manschette (15) dem Bereich des Handgelenkes angepaßt ist und über dem Handrücken mit einer die Venen (13) schützenden Brücke (16) unterlegt ist, und ein aufblasbarer Druckbalg (17) im Bereich der Arterie (14) an der Innenseite des Handgelenkes vorgesehen ist. Die Brücke (16) kann dabei eine gepolsterte halbe Röhre sein. Der Druckbalg (17) kann über ein umschaltbares Ventil (24) mit der Druckquelle (25) verbunden sein, wobei das Ventil (24) in Abhängigkeit von den Signalen des Pulsfühlers (18) getrennt ist. Schließlich kann zur Messung des systolischen Druckes das Ventil (24) derart gesteuert sein, daß es beim Auftreten von Pulssignalen kontinuierlich die Druckquelle (25) an den Druckbalg (17) anschließt und bei Ausbleiben von Pulssignalen den Druck aus dem Druckbalg (17) abläßt. Schließlich kann zur Messung des diastolischen Druckes das Ventil (24) derart gesteuert sein, daß es bei Ausbleiben von Pulssignalen kontinuierlich die Druckquelle (25) an den Druckbalg (17) anschließt und bei Auftreten von Pulssignalen den Druck aus dem Druckbalg (17) abläßt.

- 1 -

Vorrichtung zur Blutdruckmessung
_____

Die Erfindung betrifft eine Vorrichtung zur Blutdruckmessung, mit einer am Arm zu befestigenden Manschette,
die einen Pulsfühler aufweist, einer an die
Manschette angeschlossenen Druckquelle und einem
Druckmeßgerät zur Messung des Druckes in der Manschette.

Die bekannten Blutdruckmeßgeräte weisen eine am
Oberarm zu befestigende Manschette auf, mit der das
gesamte Gefäßsystem des Oberarmes abgeklemmt wird.
Als Pulsfühler dient ein Stethoskop oder ein
Mikrophon, mit dem die Geräusche der Schlagader abgehört werden. Die Manschette wird mit einer Pumpe
so stark aufgepumpt, daß die Pulsgeräusche verschwinden.
Anschließend wird Luft abgelassen, bis Pulsgeräusche
hörbar sind. Der dann in der Manschette herrschende
Druck wird gemessen. Er entspricht dem systolischen
Blutdruck. Beim weiteren Ablassen von Luft werden
die Pulsgeräusche wieder schwächer bzw. verschwinden.
Der dann in der Manschette herrschende Luftdruck
entspricht dem diastolischen Druck.

Derartige Methoden der Blutdruckmessung sind umständlich und erlauben keine kontinuierliche Überwachung durch den Arzt bzw. durch den Patienten selbst. Durch das starke Abdrücken der Venen entsteht ein Blutstau, der nicht über längere Zeit hinweg aufrechterhalten werden sollte.

Es besteht ein großes Interesse daran, den Blutdruck am Menschen kontinuierlich messen zu können. Hierzu sind bisher nur sogenannte blutige Methoden bekannt, die nur in Notfällen bei größeren Operationen und auf Intensivstationen bei stationären Patienten in lebensbedrohlichen Situationen eingesetzt werden.

Der Erfindung liegt die Aufgabe zugrunde, eine Vorrichtung zur Blutdruckmessung zu schaffen, die eine quasi-kontinuierliche Messung ermöglicht und eine dauernde Überwachung gestattet, ohne die Venen ständig abgequetscht zu halten.

Zur Lösung dieser Aufgabe ist erfindungsgemäß vorgesehen, daß die Manschette dem Bereich des Handgelenkes angepaßt ist und über dem Handrücken mit einer die Venen schützenden Brücke unterlegt ist, und daß ein aufblasbarer Druckbalg im Bereich der Arterie an der Innenseite des Handgelenks vorgesehen ist.

- 3 -

Da in den Venen ein erheblich niedrigerer Druck
herrscht als in den Arterien, werden bei der
üblichen Abquetschung des Armes zum Zwecke der Blutdruckmessung die Venen zuerst zugedrückt. Da bei
abgequetschten Venen durch die Arterie immer noch
Blut zuströmt, kommt es zum Blutstau. Erfindungsgemäß wird der Blutstau dadurch verhindert, daß
lediglich eine oder mehrere Arterien, nicht aber die
Venen, zusammengedrückt werden. Dies führt dazu, daß
keine unangenehmen Blutstaugefühle auftreten, so daß
die Manschette auch ständig getragen werden kann. Die
Manschette wird in der Nähe des Handgelenks nach Art
einer Armbanduhr befestigt. Sie bildet daher keine
größere Erschwernis oder Behinderung für den Probanden.
Der Druckbalg drückt gezielt gegen die Arterie der
Innenseite des Unterarms bzw. des Handgelenkes und
bewirkt kein unnötiges Abdrücken weiterer Blutgefäße.
Die Venen auf der Außenseite des Unterarms bzw. auf
dem Handrücken sind frei für den Rückfluß des Blutes,
so daß Stauungen in der Hand fast ganz vermieden werden.
Die Anastomosen der Venen in der Hand führen auch
das Blut von der Handinnenseite nach außen.

Es ist bekannt, die Pulsfrequenz am Finger auf einfache
Weise zu messen. Es wird rotes Licht in die Finger-

- 4 -

beere eingestrahlt und die Remission des Lichtes gemessen. Das remittierte Licht pulsiert mit der Pulsfrequenz, die angezeigt wird. Man nimmt an, daß die Kapillaren sich mit jeder Systole des Herzens stärker füllen, damit schwankt die Haemoglobinkonzentration im Gewebe. Dies verursacht die frequenzabhängige Schwankung der Remission von rotem Licht.

Es wurde nun gefunden, daß die so gemessene Fingerpulsfrequenz auch dann nicht verschwindet, wenn der Oberarm mit dem doppelten systolischen Druck in der Manschette abgequetscht wird. Daraus läßt sich schließen, daß das Gewebe unterhalb der Stauung im Arm noch mit Blut versorgt wird.

In vorteilhafter Weiterbildung der Erfindung ist die die Venen schützende Brücke eine gepolsterte halbe Röhre. Diese Brücke kann auch als Träger für die erforderlichen Ventile und Anzeigeeinrichtungen dienen, so daß die Blutdruckmeßeinrichtung nach Art einer Armbanduhr zu tragen ist.

Dies erlaubt ihren Einsatz bei der Dauerüberwachung von Sportlern, Patienten, Rehabilitanten und anderen Personen.

In vorteilhafter Weiterbildung der Erfindung ist der Druckbalg über ein umschaltbares Ventil mit der Druckquelle verbunden, und das Ventil ist in Abhängigkeit von den Signalen des Pulsfühlers gesteuert. Als Druckfühler kann ein Mikrophon oder ein anderer Umsetzer verwendet werden, der Druck in elektrische Signale verwandelt. Dadurch, daß der Pulsfühler die Drucksteuerung der Manschette übernimmt, ist gewährleistet, daß der Druckluftverbrauch so gering wie möglich gehalten wird. Außerdem werden ein übermäßiges Aufpumpen des Druckbalges und damit unnötig starke Abquetschungen der Arterie vermieden. Das Abdrücken der Arterie wird auf den für die Messung unbedingt erforderlichen Wert begrenzt.

In zweckmäßiger Weiterbildung der Erfindung ist zur Messung des systolischen Druckes das Ventil derart gesteuert, daß bei Auftreten von Pulssignalen kontinuierlich die Druckluftquelle an den Druckbalg anschließt und bei Ausbleiben von Pulssignalen den Druck aus dem Druckbalg abläßt. Zur Messung des diastolischen Druckes ist das Ventil derart gesteuert, daß es bei Ausbleiben von Pulssignalen kontinuierlich die Druckquelle an den Druckbalg anschließt und bei Auftreten von Pulssignalen den Druck aus dem Druckbalg abläßt.

- 6 -

Die erfindungsgemäße Vorrichtung ist von besonderer Bedeutung für das sogenannte Bio-Feedback. Hierbei werden vegetative Körperfunktionen, die sich durch bewußte Konzentration beeinflussen lassen, wie Hauttemperatur, Atmung und Pulsfrequenz gemessen und dem Probanden mitgeteilt. Dieser hat die Möglichkeit, durch Konzentration und innere Einstellung das normale Gleichgewicht der Körperfunktionen wieder herzustellen. Für den Blutdruck fehlte infolge des Mangels an einer einfachen Vorrichtung zur Blutdruckmessung bisher diese Möglichkeit.

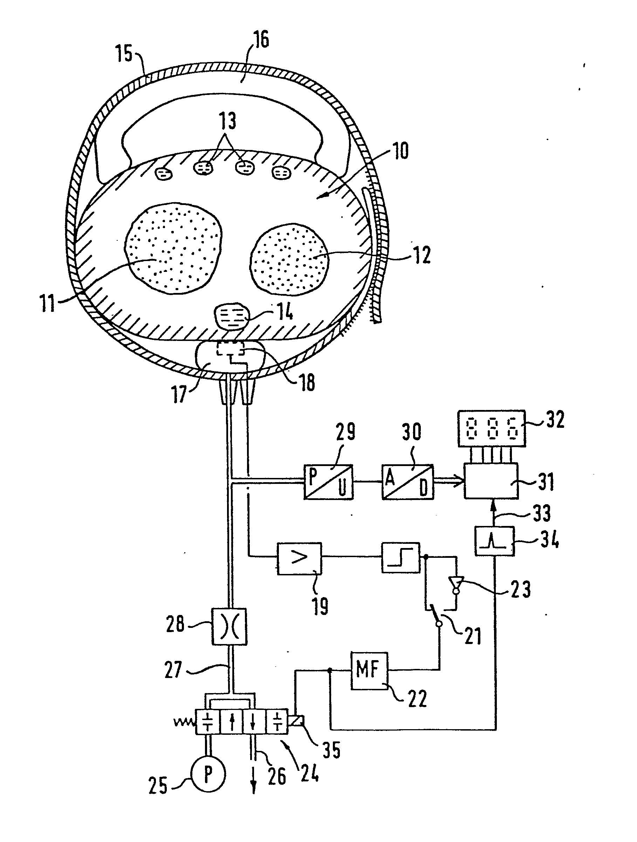

Im folgenden wird unter Bezugnahme auf die einzige Figur der Zeichnung ein Ausführungsbeispiel der Erfindung näher erläutert, welches eine bevorzugte Ausführungsform darstellt.

In der Zeichnung ist ein Querschnitt durch den Unterarm mit angelegter Manschette zusammen mit einem Blockschaltbild des pneumatischen und elektronischen Teils der Blutdruckmeßeinrichtung dargestellt.

In dem Unterarm 10 befinden sich die Knochen von Elle 11 und Speiche 12. Auf der Außenseite verlaufen die Venen 13 und auf der Innenseite der Arterie 14.

Die Vorrichtung zur Blutdruckmessung weist eine Manschette 15 auf, die um den Unterarm 10 herumgelegt wird. In der Innenseite der Manschette 15 ist eine starre, aber gepolsterte Brücke 16 angebracht, die die Venen 13 überbrückt, so daß bei gespannter Manschette 15 die Venen 13 nicht zusammengedrückt werden. Die Brücke 16 ist U-förmig ausgebildet und stützt sich mit den Enden ihrer Schenkel am Unterarm ab. Auf der der Brücke 16 abgewandten Seite der Manschette befindet sich ein aufblasbarer Druckbalg 17, der im aufgeblasenen Zustand gezielt gegen die Arterie 14 drückt. Der Druckbalg 17 enthält ein Mikrophon 18 zur Aufnahme der Pulsgeräusche der Arterie 14. Diese Pulsgeräusche werden in einem Verstärker 19 verstärkt und einer Schwellwertschaltung 20 zugeführt. Am Ausgang der Schwellwertschaltung 20 entsteht ein Signal, wenn das Eingangssignal der Schwellwertschaltung einen bestimmten Amplitudenwert übersteigt. Der Ausgang der Schwellwertschaltung 20 ist in der einen Schaltstellung eines Schalters 21 mit dem Eingang einer monostabilen Kippstufe 22 und in der anderen Stellung des Schalters 21 über einen Inverter 23 mit dem Eingang einer monostabilen Kippstufe 22 verbunden. Da die von dem Mikrophon 18 aufgenommenen Pulsschläge impulsweise auftreten, sorgt die monostabile Kippstufe 22 dafür, daß ein kontinuierliches Signal erzeugt wird, wenn bei der dargestellten Stellung des Schalters 21 die Impulse den Schwellenwert der Schwellwertschaltung 20 übersteigen. Die monostabile Kippstufe 22 hat also eine Laufdauer, die etwas größer ist als der zeitliche Abstand zweier Pulsschläge.

Der Ausgang der monostabilen Kippstufe 22 steuert ein Magnetventil 24 . Dieses verbindet eine Druckluftleitung 27 in der einen Schaltstellung mit einer Druckquelle 25 und in der anderen Schaltstellung mit einem Auslaß 26. Die Druckluftleitung 27 ist über ein Drosselelement 28 mit dem Inneren des Druckbalges 17 verbunden.

Der Druck im Druckbalg 18 wird einem Druck/Spannungs-Umsetzer 29 zugeführt, dessen Ausgang mit einem Analog/-Digital-Wandler 30 verbunden ist. Der Analog/Digital-Wandler 30 gibt ein dem Luftdruck entsprechendes Digitalsignal an ein Register 31, das mit einer digitalen Anzeigeeinrichtung 32 verbunden ist. Die Übernahme des Signals in das Register 31 wird von einer Leitung 33 gesteuert, die über ein Differenzierglied 34 mit dem Ausgang der monostabilen Kippstufe 22 verbunden ist.

- 9 -

Die Wirkungsweise der Vorrichtung ist folgende:

Nach dem Anlegen der Manschette 15 in der dargestellten Weise wird das Magnetventil 24 so eingestellt, daß die Leitung 27 kurze Zeit mit der Druckluftquelle 25 verbunden wird. Dadurch wird der Druckbalg 17 auf einen mittleren Druckwert aufgeblasen. Erst danach tritt die Steuerschaltung in Funktion. Zur Messung des systolischen Druckes wird der Schalter 21 in die dargestellte linke Stellung gebracht, in der der Ausgang der Schwellwertschaltung 20 direkt mit dem Eingang der monostabilen Kippstufe 22 verbunden ist. Werden am Mikrophon 18 Pulsgeräusche aufgenommen, dann wird jedesmal die Ansprechschwelle der Schwellwertschaltung 20 überschritten und die monostabile Kippstufe 22 wird bei jedem Pulsschlag von neuem angestoßen. Sie liefert also ein kontinuierliches Ausgangssignal an den Steuermagneten 35 des Magnetventils 24. Dieses wird eingeschaltet und verbindet die Druckluftquelle 25 mit dem Druckbalg 17. Wenn daraufhin die Pulsgeräusche schwächer werden, spricht die Schwellwertschaltung 20 nicht mehr an, so daß das Ausgangssignal der monostabilen Kippstufe 22 abfällt und der Elektromagnet 35 aberregt wird. Die Rückflanke des Signals der monostabilen Kippstufe 22 steuert über das Differenzierglied 34 die Eingabe
des entsprechenden Druckwertes in das Register 31,
so daß der jeweilige Augenblickswert an der Anzeigeeinrichtung 32 zur Anzeige gebracht wird. Dieser
Wert stellt den systolischen Druck dar. Das Magnetventil 24 kehrt in seine Ruhelage zurück, in der die
Leitung 27 mit dem Auslaß 26 verbunden ist, so daß
die Druckluft aus dem Druckbalg 17 über das Drosselelement 28 langsam entweicht.

Zur Messung des diastolischen Druckes wird der
Schalter 21 in die rechte Schalterstellung gebracht.
Ausgehend von dem voreingestellten mittleren Luftdruck wird nun, wenn die Pulssignale die Schaltschwelle
des Schwellwertschalters 20 übersteigen, der
Elektromagnet 35 aberregt, und das Magnetventil 24
in die Ruhestellung gebracht, in der der Druck aus
der Leitung 27 und dem Druckbalg 17 langsam abgelassen wird. Wenn daraufhin die Amplituden der Pulssignale unter den Schwellwert der Schwellwertschaltung
20 absinken, wird die monostabile Kippstufe 22 angestoßen und das Magnetventil 24 wird in die Arbeitsstellung geschaltet, in der Druck aus der Druckluftquelle 25 in den Druckbalg 17 nachgepumpt wird.
Bei diesem Umschaltvorgang spricht die Differenzier-

- 11 -

schaltung 34 an, die daraufhin über Leitung 33 die
Eingabe des betreffenden Druckwertes in das Register
31 veranlaßt. An der Anzeigeeinrichtung 32 wird der
diastolische Druck angezeigt.

Als Druckquelle 25 kann eine Druckgaspatrone oder auch
eine motorgetriebene Luftpumpe verwendet werden. Für
mobile Geräte, die insgesamt am Patienten angebracht
werden, empfiehlt sich eine Druckgaspatrone.

- 1 -

A n s p r ü c h e
———————————————————

1. Vorrichtung zur Blutdruckmessung mit einer am Arm zu befestigenden Manschette, die einen Pulsfühler aufweist, einer an die Manschette angeschlossenen Druckquelle und einem Druckmeßgerät zur Messung des Druckes in der Manschette, dadurch gekennzeichnet, daß die Manschette (15) dem Bereich des Handgelenkes angepaßt ist und über dem Handrücken mit einer die Venen (13) schützenden Brücke (16) unterlegt ist, und daß ein aufblasbarer Druckbelag (17) im Bereich der Arterie (14) an der Innenseite des Handgelenkes vorgesehen ist.

2. Vorrichtung nach Anspruch 1, dadurch gekennzeichnet, daß die Brücke (16) eine gepolsterte halbe Röhre ist.

3. Vorrichtung nach Anspruch 1 und 2, dadurch gekennzeichnet, daß der Druckbalg (17) über ein umschaltbares Ventil (24) mit der Druckquelle (25) verbunden ist, und daß das Ventil (24) in Abhängigkeit von den Signalen des Pulsfühlers (18) getrennt ist.

4. Vorrichtung nach Anspruch 3,

dadurch gekennzeichnet,

daß zur Messung des systolischen Druckes das Ventil (24) derart gesteuert ist, daß es bei Auftreten von Pulssignalen kontinuierlich die Druckquelle (25) an den Druckbalg (17) anschließt und bei Ausbleiben von Pulssignalen den Druck aus dem Druckbalg (17) abläßt.


5. Vorrichtung nach Anspruch 3,

dadurch gekennzeichnet,

daß zur Messung des diastolischen Druckes das Ventil (24) derart gesteuert ist, daß es bei Ausbleiben von Pulssignalen kontinuierlich die Druckquelle (25) an den Druckbalg (17) anschließt und bei Auftreten von Pulssignalen den Druck aus dem Druckbalg (17) abläßt.

0041696

1/1

Europäisches
Patentamt

**EUROPÄISCHER RECHERCHENBERICHT**

EP 81 10 4284

| Kategorie | EINSCHLÄGIGE DOKUMENTE | | KLASSIFIKATION DER ANMELDUNG (Int. Cl.³) |
|---|---|---|---|
| | Kennzeichnung des Dokuments mit Angabe, soweit erforderlich, der maßgeblichen Teile | betrifft Anspruch | |
| | US - A - 3 104 661 (W. HALPERN/ BECKMAN INSTRUMENT, INC.)<br><br>* Spalte 2, Zeilen 15-35; Spalte 7, Zeilen 39-56; Abbildung 4 *<br><br>-- | 1 | A 61 B 5/02 |
| | US - A - 3 621 831 (J.S. PISACANO)<br><br>* Spalte 1, Zeilen 44-62; Spalte 2, Zeilen 13-59; Abbildungen 1-6 *<br><br>-- | 1 | |
| | US - A - 3 757 772 (A. GOLDBLAT + R. MILLER)<br><br>* Zusammenfassung; Spalte 3, Zeile 40 - Spalte 5, Zeile 42; Abbildungen 1-7 *<br><br>-- | 1 | **RECHERCHIERTE SACHGEBIETE (Int. Cl.³)**<br><br>A 61 B 5/02 |
| | GB - A - 2 023 849 (MATSUSHITA ELECTRIC WORKS LTD.)<br><br>* Zusammenfassung; Seite 2, Zeile 59 - Seite 3, Zeile 107; Seite 4, Zeile 105 - Seite 5, Zeile 37; Abbildungen 2,3,8 *<br><br>& WO - A - 79/00294 | 1,3,5 | |
| P | & EP - A - 0 014 720<br><br>-- | | |
| | DE - B - 1 013 036 (K. BRECHT + B. BOUCKE)<br><br>* Spalte 3, Zeile 57 - Spalte 4, Zeile 16; Abbildungen 1,2 *<br><br>---- | 1,3 | |

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 15-09-1981 | RIEB |

EPA form 1503.1   06.78